Europäisches Patentamt

European Patent Office

Office européen des brevets

(19)

(11) Veröffentlichungsnummer: **0 043 826**
**B1**

(12) **EUROPÄISCHE PATENTSCHRIFT**

(45) Veröffentlichungstag der Patentschrift:
**27.12.84**

(21) Anmeldenummer: **81900221.3**

(22) Anmeldetag: **10.01.81**

(86) Internationale Anmeldenummer:
**PCT/EP 81/00001**

(87) Internationale Veröffentlichungsnummer:
**WO 81/02065 (23.07.81** Gazette **81/17)**

(51) Int. Cl.³: **G 01 N 33/00,** G 01 N 27/72,
G 01 N 1/22

(54) **VORRICHTUNG ZUR BESTIMMUNG DES MAGNETIT- UND PHOSHORGEHALTES VON ERZEN.**

(30) Priorität: **18.01.80 DE 3001704**

(43) Veröffentlichungstag der Anmeldung:
**20.01.82 Patentblatt 82/3**

(45) Bekanntmachung des Hinweises auf die Patenterteilung:
**27.12.84 Patentblatt 84/52**

(84) Benannte Vertragsstaaten:
**AT CH DE FR GB LI LU NL SE**

(56) Entgegenhaltungen:
**GB - A - 599 613**
**LU - A - 53 954**
**US - A - 3 137 543**
**US - A - 3 686 563**
**US - A - 3 808 524**
**US - A - 3 843 198**

(73) Patentinhaber: **LUOSSAVAARA-KIIRUNAVAARA AB,**
**Sturegatan 11, S-100 41 Stockholm (SE)**

(72) Erfinder: **KÖNIG, Rainer, Dörnweg 13, D-6236 Eschborn**
**(DE)**
Erfinder: **SIEGLEN, Rolf, Im Hohlweg 8a,**
**D-6231 Sulzbach (DE)**
Erfinder: **WEISSER, Wolfgang,**
**Graf-Staufenberg-Ring 82, D-6380 Bad Homburg v.d.H.**
**(DE)**
Erfinder: **HEIDE, Helga, Am Hohenstein 14,**
**D-6233 Kelkheim (DE)**

(74) Vertreter: **Rupprecht, Klaus, Dipl.-Ing., Am Römerhof 35,**
**D-6000 Frankfurt (Main) 90 (DE)**

## Beschreibung

Die Erfindung betrifft eine Vorrichtung zur Bestimmung des Magnetit- und Phosphorgehaltes von Erzen, mit einer magnetfelderzeugenden Messspule zur Bestimmung des Magnetitgehalts und einem Probebehälter, der im Kern der Messspule angeordnet ist.

Beim Abbau mineralischer Rohstoffe muss die Rohstoffzusammensetzung ständig kontrolliert werden. Zum einen wird dabei der Anteil von Taubgestein beziehungsweise Nutzgestein bestimmt, zum andern ist es oft erforderlich, bestimmte störende Beimengungen unter Kontrolle zu halten. Üblicherweise werden dabei stichprobenartige Mengen des Rohstoffes mit Hilfe einer Schaufel oder mit anderen Greifwerkzeugen entnommen. Die Probenmenge wird dann für die eigentliche chemische Analyse aufbereitet, z.B. gemahlen und homogenisiert. Die Analyse muss in so kurzer Zeit durchgeführt werden, dass in Abhängigkeit von dem Analysenergebnis in den Weitertransport oder in die Weiterverarbeitung der Materialien eingegriffen werden kann.

Da die gesamte Analyse einschliesslich der Probenahme und Aufbereitung relativ lange dauert, ist es bisher nur möglich, in viel zu grossen Zeitabständen Proben zu entnehmen und in Abhängigkeit von den Analysenergebnissen in den Prozess einzugreifen. So hat dies z.B. beim Abbau von Eisenerz in einer grossen Untertagemine eine ungewollte Aufmischung von Erzen verschieden hoher Phosphorgehalte zur Folge. Diese Aufmischung führt dazu, dass die Ausbeute an phosphorarmem Erz erheblich unter der vom geologischen Standpunkt aus errechneten Mengen liegt. Die Aufmischung geschieht bereits beim Abbau vor Ort. Das abgesprengte Erz wird mit Hilfe von Schaufelladern zu ca. 40 bis 200 m entfernten Schächten transportiert. Der Inhalt einer Schaufel beträgt ungefähr 8 bis 14 t. Nur von etwa jeder zehnten Schaufel wird mit Hilfe eines Greifwerkzeuges eine ca. 1 kg schwere Probe entnommen. Diese Probe wird gemahlen, gesiebt, magnetisch separiert und davon eine Teilmenge getrocknet, davon wiederum eine Teilmenge abgemessen und diese nasschemisch analysiert. Aufgrund dieses Analysenergebnisses werden bis zum Vorliegen des nächsten Analysenergebnisses alle weiteren Schaufeln in denjenigen Schacht gekippt, der für die analysierte Qualitätsstufe vorgesehen ist, obwohl sich in der Regel die Zusammensetzung des Materials von Schaufel zu Schaufel ändert. Im Phosphorgehalt, der die Qualität wesentlich bestimmt, können Schwankungen um den Faktor 10 bis 100 auftreten.

Aus LU-A 53 957 ist ein Verfahren zur Bestimmung des Magnetitgehaltes von Erzen bekannt, bei welchem eine Probe in ein elektromagnetisches Feld gebracht und aus der Änderung des magnetischen Flusses bzw. der relativen Permeabilität der Magnetitgehalt der Probe bestimmt wird. In der US-A 3 137 543 wird ein Verfahren beschrieben, bei dem der Phosphoranteil der gesamten Probenmenge ermittel wird. Ferner ist aus US-A 3 843 198 eine Vorrichtung bekannt, mit der zum Zwecke der Analyse durch ein Schneidrad Gesteinspartikel erzeugt und aufgesaugt werden können.

Der vorliegenden Erfindung liegt nun die Aufgabe zugrunde, eine Vorrichtung zu entwickeln, mit dem in rascher Folge repräsentative Proben des Materials, das analysiert und sortiert weitergeleitet werden soll, entnommen und in ausreichend kurzer Zeit auf Magnetit- und Phosphorgehalt analysiert werden können. Mit einem solchen Gerät soll beim Erzabbau in der zuvor beschriebenen Weise jeder Schaufel eine Probe entnommen und so schnell analysiert werden, dass ohne Wartezeit der Schaufelinhalt in den für diese Zusammensetzung beziehungsweise für diese Qualität vorgesehenen Schacht geleitet werden kann.

Diese Aufgabe ist erfindungsgemäss dadurch gelöst, dass der minimale Füllstand des Probenbehälters höher ist als die Messspule und dass zur Bestimmung der Probenmenge eine weitere Messspule vorgesehen ist, deren Länge grösser ist als die maximale Füllstandshöhe des Probenbehälters und dass der Probenbehälter mit einem Reaktionsgefäss zur Durchführung der Phosphoranalyse verbunden ist und das Reaktionsgefäss mit Reagens-Vorratsbehältern in Verbindung steht. Vorteilhafte Ausführungsformen der erfindungsgemässen Vorrichtung sind in den Unteransprüchen 2 bis 10 beschrieben.

Mit einer derartigen Einrichtung wird eine Probe aus künstlich erzeugtem oder als Nebenprodukt anfallendem Staub gesammelt und in ein elektromagnetisches Feld gebracht. Aus der Änderung des magnetischen Flusses beziehungsweise der relativen Permeabilität wird der Magnetitgehalt der Probe bestimmt und anschliessend wird der Phosphoranteil der gesamten Probenmenge ermittelt. Die Gesamtprobenmenge wird ebenfalls aus der Änderung des magnetischen Flusses beziehungsweise der relativen Permeabilität bestimmt.

Zur Gewinnung eines möglichst repräsentativen Probematerials wird ein Staubsammelteil verwendet, dessen spezielle Konstruktion insbesondere folgende Merkmale in sich vereinigt:
- Unterdrückung der Feinstaubfraktion gegenüber der Grobstaubfraktion, um eine gut rieselfähige und in ihre Schüttdichte gut reproduzierbare Probe erhalten zu können.
- Einfache Überführung des abgeschiedenen Grobstaubs zu einem Behälter, in dem die Masse der Staubprobe sowie der Magnetitgehalt bestimmt werden.

Das Staubsammelteil ist als eine z.B. zylindrische Saugeinrichtung mit seitlichem Saugstutzen ausgebildet. Die Staubprobe wird zunächst durch einen Vorfilter mit einer Maschenweite von z.B. 100, $\mu$m angesaugt. Die Einrichtung ist innen mit einem koaxial angeordneten Siebeinsatz beziehungsweise einer Filtergaze versehen. Die Länge und der Durchmesser des durch den Filtereinsatz abgegrenzten Hohlzylinders ist mit der Maschenweite des Filtereinsatzes so abgestimmt, dass alle Partikel, die grösser als die Maschenweite des Filters sind, den Behälter zum Sammeln des Staubs erreichen, da sie aufgrund ihrer Trägheitskräfte nicht genügend abgebremst und umgelenkt werden. Die kleineren Partikel dagegen werden durch die Maschen des Sieb- beziehungsweise Filtereinsatzes abgesaugt. Ferner ist die Saugeinrichtung vorne mit einer düsenartigen Verengung versehen, wodurch die durch den Vorfilter angesaugten Staubpartikel so stark beschleunigt werden, dass die grösseren Partikel der durch die seitliche Absaugung erzielten Umlenkung des Luftstroms nicht folgen.

Die Einrichtung zur Analyse des Staubes auf Magnetit- beziehungsweise Phosphorgehalt besteht im wesentlichen aus zwei Hauptkomponenten: einer Magnetspuleneinheit zur Bestimmung des Magnetitgehaltes sowie der Gesamtprobenmenge und einer Einrichtung zur nasschemischen Analyse von Phosphor. In der Magnetspuleneinheit ist ein länglicher Behälter vorgesehen, der zur Aufnahme des zu analysierenden Probenmaterials dient. Dieser Behälter ist von einer Spulenanordnung umgeben. Wird die Spulenanordnung an eine Wechselspannung angelegt, entsteht im Innern der Spule ein magnetisches Feld. Durch Einbringen der zu vermessenden Probe in den im Spulenkern angeordneten rohrförmigen Behälter wird der magnetische Fluss beziehungsweise die relative Permeabilität des Kerns in Abhängigkeit von dem Magnetitgehalt der Probe geändert. Aus dieser Änderung lässt sich sowohl die Füllstandshöhe beziehungsweise die Gesamtmenge der Probe als auch der Magnetitgehalt der Probe bestimmen.

Bei Verwendung von zwei koaxial angeordneten Spulen dient die Spule, deren Länge kleiner als die minimale Füllstandshöhe des Behälters ist, zur Messung des Magnetitgehaltes und die zweite Spule, deren Länge grösser als die maximale Füllstandshöhe des Behälters ist, zur Messung der Gesamtprobenmenge.

Besteht die Spulenanordnung aus drei Spulen, so wird eine Spule als Primärspule verwendet. Ihre Länge ist grösser als die maximale Füllstandshöhe der die Probe enthaltenden Säule. Bei vorgegebener Primärspannung $U_P$ ist die Sekundärspannung $U_1$ eine Funktion des Magnetitgehaltes der Staubprobe. Aus dem Verhältnis der Spannungen $U_2/U_1$ lässt sich ausserdem die Füllstandshöhe der magnetithaltigen Staubsäule bestimmen. Dabei gelten im einzelnen folgende Beziehungen:

$$C_V^M = f(U_1) \qquad (I)$$

wobei $C_V^M$ der prozentuale Volumenanteil des Magnetits bedeutet. Ist die Masse der Gesamtprobe $M_D$, so gilt

$$M_D = f(U_2)\,(1 - \gamma_W \cdot \gamma_M^{-1}) + \frac{f(U_2)}{f(U_1)} \cdot \gamma_M^{-1} \cdot \gamma_W \qquad (II)$$

wobei

$$f(U_1) = k_2\,U_1 + k_3 = C_V^M \qquad (III)$$

$$f(U_2) = k_1\,\frac{U_2}{U_1} \text{ und } \Delta\gamma = \gamma_M - \gamma_W \qquad (IV)$$

sind und $\gamma_M$ spezifisches Gewicht von Magnetit und $\gamma_W$ spezifisches Gewicht des nichtmagnetithaltigen Restes der Probe bedeuten.

Für die Masse der Gesamtprobe ergibt sich dann:

$$M_D = \Delta\gamma \cdot k_1 \left( k_2\,U_2 + k_3\,\frac{U_2}{U_1} \right) + \gamma_W \cdot k_1\,\frac{U_2}{U_1} \qquad (V)$$

Für eine genaue Bestimmung des Magnetitgehaltes beziehungsweise des Eisengehaltes muss bei gleichbleibender, geringer Feuchtigkeit eine homogene und weitgehend gleiche Korngrössenverteilung in der Staubprobe gewährleistet sein, da unterschiedliche Schüttdichten der Probe in der Säule die Messgenauigkeit negativ beeinflussen.

Die Staubprobe, deren Masse durch die Magnetspulenanordnung bestimmt worden ist, wird auf Phosphor analysiert. Die Messung des Phosphorgehaltes erfolgt dabei in an sich bekannter Weise photometrisch, wobei die Probe mit einem Molybdat/Vanadat-Reagenz umgesetzt wird. Hierfür muss die Reagenz-Säurekombination genau dosiert werden. Später erfolgt in analoger Weise die Dosierung von Wasser zur Verdünnung. Die Einleitung der Reagenzflüssigkeit beziehungsweise Wasser in die Reaktionskammer erfolgt durch eine spezielle Anordnung von Vorratsbehältern und Dosierkammern sowie Ventilen. Nach der Durchführung der Analyse werden die Messwerte in einem Mikrocomputer ausgewertet. Der Mikrocomputer kann gleichzeitig die Ablaufsteuerung der Anlage übernehmen.

Die durch die Erfindung erzielten Vorteile sind im wesentlichen darin zu sehen, dass genügend grosse Staubmengen für die Analyse bereitgestellt werden können. Zusätzlich wird ermöglicht, unterschiedliche Staubmengen genau zu messen, was die Voraussetzung für die Phosphoranalyse ist. Ferner ist die Vorrichtung auf einen Lader montierbar und hält gegenüber den hohen Belastungen, die beim Ladeprozess entstehen, stand. Die nasschemische Analysenanlage für Phosphor ist so konzipiert, dass ausser einigen Magnetventilen keinerlei bewegliche Teile vorhanden sind, so dass sie den Erfordernissen nach einem erschütterungsunempfindlichen und einfachen Analysegerät entspricht. Sowohl die Magnetitgehaltbestimmung als auch die Phosphorbestimmung lässt sich in einer sehr kurzen Zeit von weniger als 60 sec durchführen. Für besondere Anwendungszwecke können die elektronischen Komponenten der Vorrichtung auch ausserhalb des Gerätekörpers untergebracht werden, ohne dass die Funktion beeinträchtigt wird.

Im folgenden wird die Erfindung anhand von lediglich einen Ausführungsweg darstellenden Zeichnungen näher erläutert. Es zeigen in schematischer Vereinfachung

Figur 1 den Aufbau der Gesamtvorrichtung;
Figur 2 im Querschnitt das Staubsammelteil
Figur 3 eine mögliche Form der Stauberzeugung und
Figur 4 den Aufbau der Analyseeinrichtung für Phosphor.

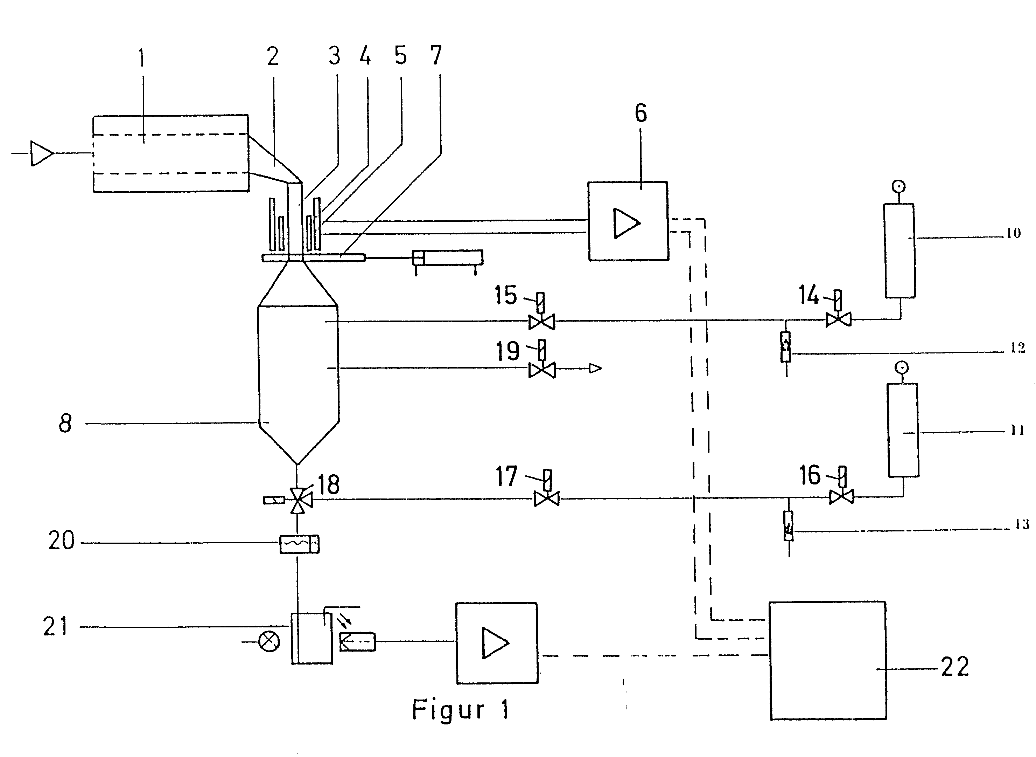

Aus Figur 1 geht hervor, dass das Staubsammelteil 1 über eine Leitung 2 mit einem länglichen Behälter 3 verbunden ist. Der Behälter 3 ist in diesem Ausführungsbeispiel von zwei Spulen 4 und 5 umgeben, die von einer Wechselstromquelle 6 gespeist werden.

Die durch das Staubsammelteil 1 geförderten Partikel der Korngrösse im Bereich von 100 bis 15 μm, vorzugsweise eines mittleren Massedurchmessers von 65 μm, können bei ca. 100°C getrocknet werden, indem man an der Leitung 2 und/oder am Behälter 3 eine – hier nicht gezeigte – Heizeinrichtung vorsieht. Die Längen der Spulen 4 und 5 sind mit der Füllhöhe des Behälters 3 in der Weise abgestimmt, dass die Spule 4 kürzer, aber die Spule 5 länger ist als die Staubsäule. Die genaue Messung der Füllhöhe kann dann durch die Spule 5 erfolgen, während die Spule 4 zur Bestimmung des Magnetitgehaltes dient. Aus den Messwerten der Spulen 4 und 5 lässt sich die Gesamtmasse der Probe berechnen.

Nach Abschluss der magnetischen Messung, die in wenigen Sekunden beendet ist, wird der Schieber 7 geöffnet und die Probe fällt in das Reaktionsgefäss 8. Hier wird die für die photometrische Analyse erforderliche Reaktion des Phosphors mit saurem Molybdat/Vanadat-Reagenz bei ca. 90°C eingeleitet. Die Dosierung der Säure/Reagenz-Kombination sowie des Wassers zur Verdünnung erfolgt unter Verwendung von Vorratsbehältern 10, 11, Dosierkammern 12, 13 und Ventilen 14 bis 18. Mit Hilfe von Pressluft, die über das Ventil 19 eingeleitet wird, erfolgt eine kräftige Verwirbelung des flüssigen Mediums mit dem Staub und führt zur raschen Auflösung der phosphorhaltigen Komponente. Liegt die Phosphorverbindung z.B. in Form von Apatit vor, so ist mit einer optimalen Zusammensetzung der Säure/Reagenz-Kombination die Reaktion innerhalb von 30 sec abgeschlossen. Danach wird über den Filter 20 die Lösung in eine Durchflussküvette mit Photometereinheit 21 geleitet. Die Messwertauswertung erfolgt durch einen Mikrocomputer 22, der gleichzeitig die Ablaufsteuerung der Anlage übernehmen kann.

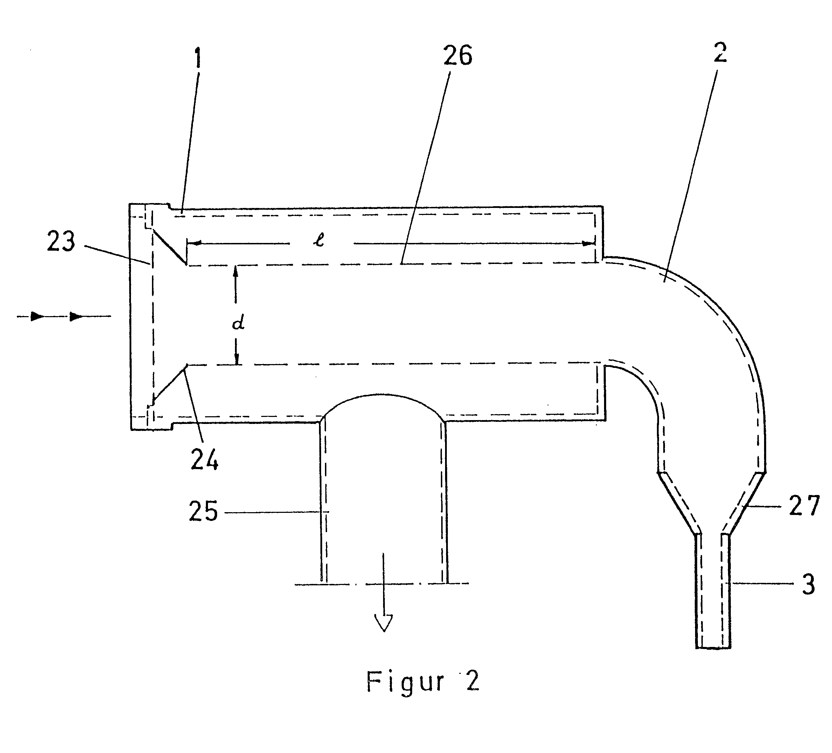

Aus Figur 2 wird das Funktionsprinzip des erfindungsgemässen Staubsammelteils ersichtlich. Die Ansaugung erfolgt durch den Vorfilter 23, der eine Maschenweite von ca. 100 μm besitzen kann. Dadurch gelangen nur Korngrössenfraktionen von weniger als 100 μm in die hier als zylindrisches Rohr ausgebildete Saugeinrichtung 1. Die am vorderen Teil befindliche konische Verjüngung 24 der Saugeinrichtung verursacht eine starke Beschleunigung der Staubpartikel. Die Luft wird durch den seitlichen Saugstutzen 25 abgesaugt. Die Saugeinrichtung ist innen mit einem koaxial angeordneten Siebeinsatz beziehungsweise Filtergaze 26 versehen. Dieser Siebeinsatz sorgt aufgrund seines Strömungswiderstandes für eine gleichmässige Druckverteilung in der Saugeinrichtung 1. Die Länge 1 und der Durchmesser d des durch die Filtergaze 26 abgegrenzten Hohlzylinders ist mit der Maschenweite der Filtergaze so abgestimmt, dass alle Partikel, die grösser als die Maschenweite sind, die gekrümmte Leitung 2 erreichen, da sie aufgrund ihrer Massenträgheit nicht genügend abgebremst und umgelenkt werden. Die kleineren Partikel dagegen werden durch die Maschen der Filtergaze 26 gesaugt. Daraus ergibt sich, dass die Filtergaze 26 nur wenig verschmutzt wird und dadurch lange Standzeiten hat. Staubpartikel, die die gekrümmte Leitung 2 erreichen, enthalten keinen Feinanteil mehr. Das erhaltene Probenmaterial ist daher gut rieselfähig und fällt über den Trichtereinsatz 27 in den Messbehälter 3. Dieser Vorgang kann durch einen – hier nicht näher gezeichneten – Vibrator unterstützt werden. Im allgemeinen jedoch ist eine solche Massnahme nicht erforderlich, da eine genügende Vibration durch die Verwendungsweise der gesamten Vorrichtung, z.B. Montage auf einen Lader, gegeben ist. Der Trichter 27 und der Messbehälter 3 können auch ohne die gekrümmte Leitung 2 direkt an das Gehäuse der Saugeinrichtung 1 angebracht werden.

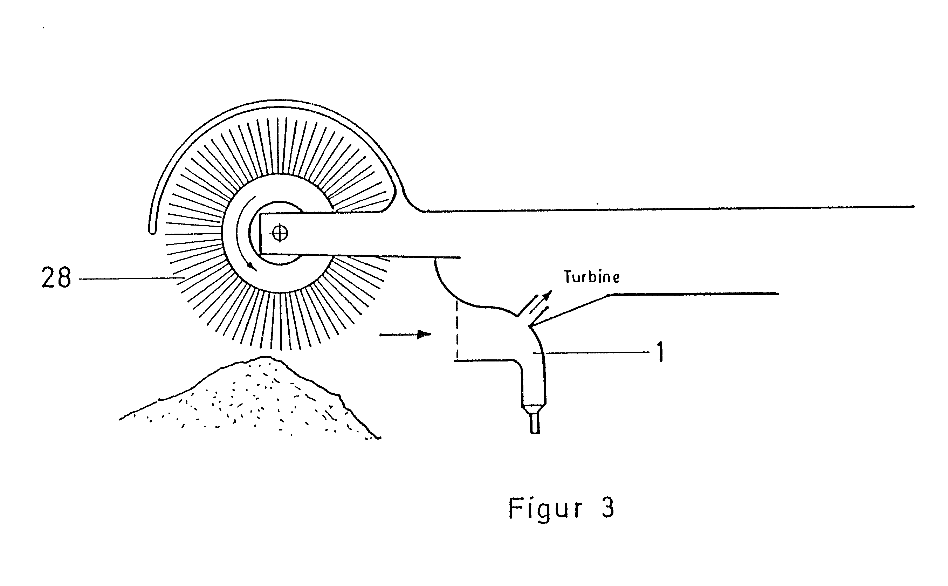

In Figur 3 wird schematisch eine Einrichtung zur Stauberzeugung dargestellt. Sie besteht im wesentlichen aus einer motorbetriebenen Ringstahlbürste 28 und kann vor dem Staubsammelteil 1 so angebracht werden, dass die Partikel von dem Staubsammelteil erfasst werden können. Eine andere Möglichkeit zur künstlichen Stauberzeugung besteht z.B. in Anwendung von Pressluft.

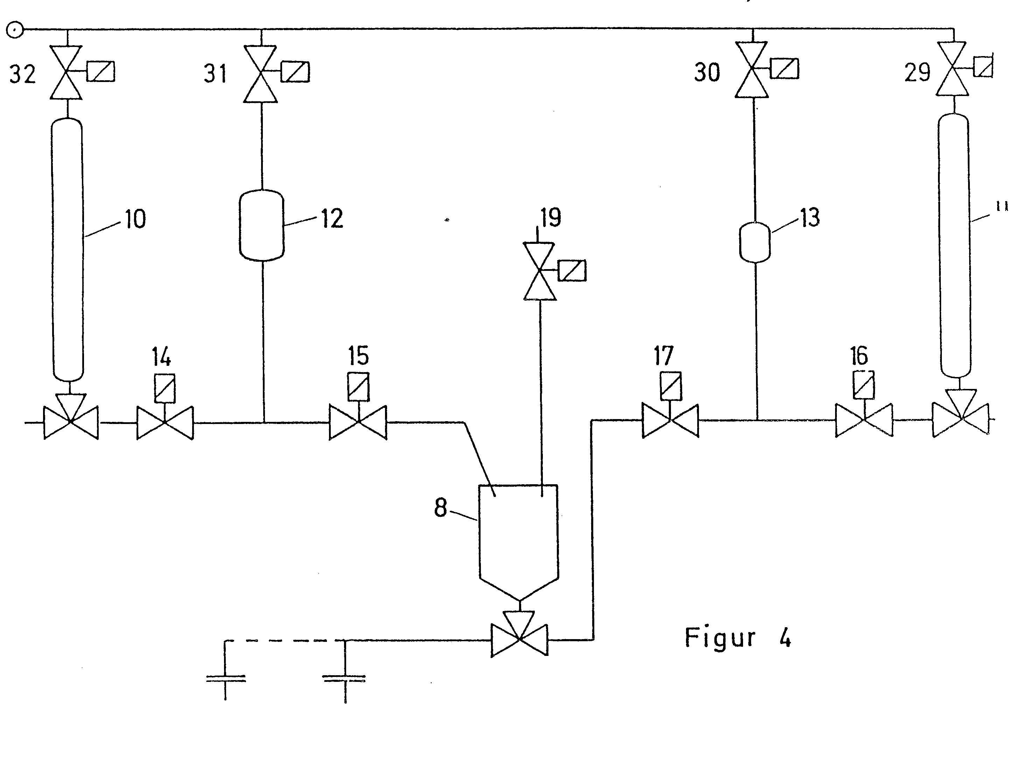

In Figur 4 wird die Funktionsweise der Einrichtung zur nasschemischen Analyse von Phosphor detaillierter dargestellt. Diese Ausführungsform dient zur zweistufigen Dosierung von zunächst einem Gemisch von Salpetersäure und Molybdat/Vanadat-Reagenz und in der zweiten Stufe der Verdünnung mit Wasser. Es ist aber auch möglich, dass nur ein Gemisch von Salpetersäure und Molybdat/Vanadat-Reagenz zudosiert wird und keine weitere Verdünnung mit Wasser erfolgt. Bei offenem Ventil 19 und 17 wird zunächst die Dosierkammer 13 entlüftet. Danach wird das Ventil 17 geschlossen, die Ventile 16 und 29 geöffnet. Dadurch wird das Vorratsgefäss 11 unter Überdruck gesetzt und es erfolgt solange ein Flüssigkeitstransport vom Vorratsbehälter 11 zur Dosierkammer 13, bis in der Dosierkammer 13 durch Kompression der Druck erreicht wird, der dem in dem Vorratsgefäss 11 angelegten Überdruck entspricht. Nach Schliessen des Ventils 16 wird das Ventil 17 und kurz danach das Ventil 30 geöffnet und es erfolgt ein Flüssigkeitstransport in die Reaktionskammer 8. Gerührt wird anschliessend mit der über das Ventil 19, Dosierkammer 13 und Ventil 17 einströmenden Pressluft. Die Zudosierung von Wasser erfolgt in analoger Weise durch die Betätigung der Ventile 14, 15, 31 und 32 von dem Vorratsgefäss 10 zu der Dosierkammer 12 und dem Reaktionsgefäss 8.

Nach Schliessen des Ventils 19 wird die Reaktionskammer 8 unter Überdruck gesetzt und die Flüssigkeit aus der Reaktionskammer 8 zur anschliessenden photometrischen Messung über das Filter 20 abfiltriert.

**Patentansprüche**

1. Vorrichtung zur Bestimmung des Magnetit- und Phosphorgehalts von Erzen mit einer magnetfelderzeugenden Messspule (4) zur Bestimmung des Magnetitgehalts und einem Probenbehälter (3), der im Kern der Messspule (4) angeordnet ist, dadurch gekennzeichnet, dass der minimale Füllstand des Probenbehälters (3) höher ist als die Messspule (4), dass zur Bestimmung der Probenmenge eine weitere Messspule (5) vorgesehen ist, deren Länge grösser ist als die maximale Füllstandhöhe des Probenbehälters (3) und dass der Probenbehälter (3) mit einem Reaktionsgefäss (8) zur Durchführung einer Phosphoranalyse verbunden ist, das mit Reagens-Vorratsbehältern (10, 11) in Verbindung steht.

2. Vorrichtung nach Anspruch 1, dadurch gekennzeichnet, dass die Spulenanordnung aus den Messspulen (4, 5) nach dem Prinzip eines Übertragers aufgebaut ist und zusätzlich zu den beiden Messspulen (4, 5) eine Primärspule aufweist, wobei die Länge der Primärspule grösser ist als die maximale Füllstandshöhe des Probenbehälters (3).

3. Vorrichtung nach Anspruch 1 oder 2, dadurch gekennzeichnet, dass zwischen den Reagensvorratsbehältern (10, 11) und dem Reaktionsgefäss (8) Dosiereinrichtungen vorgesehen sind, die aus Dosierkammern (12, 13) und entsprechenden Ventilen (14 bis 17 und 29 bis 32) bestehen, und in den Vorratsbehältern (10, 11) über Ventile (29, 32) ein Überdruck herstellbar ist.

4. Vorrichtung nach einem der Ansprüche 1 bis 3, dadurch gekennzeichnet, dass zur Messwertauswertung ein Mikrocomputer (22) vorgesehen ist, der gleichzeitig die Ablaufsteuerung der Vorrichtung übernimmt.

5. Vorrichtung nach einem der Ansprüche 1 bis 4, dadurch gekennzeichnet, dass der Probenbehälter (3) mit einer Staubsammeleinrichtung (1), vorzugsweise über eine senkrecht gekrümmte Leitung (2), in Verbindung steht.

6. Vorrichtung nach Anspruch 5, dadurch gekennzeichnet, dass an der gekrümmten Leitung (2) und/oder am Probenbehälter (3) eine Heizeinrichtung vorgesehen ist.

7. Vorrichtung nach Anspruch 5 oder 6, dadurch gekennzeichnet, dass die Staubsammeleinrichtung (1) mit einem seitlichen Saugstutzen (25) und innen mit einem koaxial angeordneten Siebeinsatz (26) versehen und vorne durch ein Vorfilter (23) abgeschlossen ist.

8. Vorrichtung nach einem der Ansprüche 5 bis 7, dadurch gekennzeichnet, dass die Staubsammeleinrichtung (1) vorne nach innen konisch verjüngt (24) ist.

9. Vorrichtung nach einem der Ansprüche 5 bis 8, dadurch gekennzeichnet, dass die Länge und der Durchmesser des durch den Siebeinsatz (26) abgegrenzten Teils der Saugeinrichtung (1) mit der Maschenweite des Siebeinsatzes (26) derart abgestimmt ist, dass Partikel, die grösser als die Maschenweite sind, den Probebehälter (3) erreichen.

10. Vorrichtung nach einem der Ansprüche 5 bis 9, dadurch gekennzeichnet, dass die Staubsammeleinrichtung (1) vorne mit einer motorbetriebenen und stauberzeugenden Ringbürste (28) versehen ist.

**Claims**

1. Device for the determination of the magnetite and phosphorus contents of ores with a measuring coil (4) generating a magnetic field for the determination of the magnetite contents and a sample-tube (3) arranged within the core of the measuring coil (4) characterized in that the minimum filling height of the sample-tube (3) exceeds the length of the measuring coil (4) and that an additional measuring coil (5) is provided for the determination of the sample mass which has a length that exceeds the maximum filling height of the sample-tube (3) and that the sample-tube (3) is connected to a reaction chamber (8) for carrying out a phosphorus analysis, and the reaction chamber is connected to reagent storage containers (10, 11).

2. Device as claimed in claim 1 wherein the coil arrangement consisting from measuring coils (4, 5) operates on the transducer principle and is provided with a primary coil in addition to both measuring coils (4, 5), the length of the primary coil exceeding the maximum filling height of the sample-tube (3).

3. Device as claimed in claim 1 or 2 wherein dosing means are provided between the reagent storage containers (10, 11) and the reaction chamber (8) which consist of dosing chambers (12, 13) and corresponding valves (14 to 17 and 29 to 32) and wherein an excess pressure can be generated in the storage container (10, 11) via the valves (29, 32).

4. Device as claimed in any of the claims 1 to 3 wherein a microprocessor (22) is provided for evaluating the measured values and simultaneously controlling the operating of the device.

5. Device as claimed in any of the claims 1 to 4 wherein the sample-tube (3) is connected to a dust collecting unit (1), preferably by means of a vertically curved piping (2).

6. Device as claimed in claim 5 wherein the curved piping (2) and/or the sample-tube (3) is provided with a heating device.

7. Device as claimed in claim 5 or 6 wherein the dust collecting unit (1) is provided with a lateral suction pipe socket (25) and a coaxial sieve insert (26) and the front of the dust collecting unit is covered with a front filter (23).

8. Device as claimed in any of the claims 5 to 7 wherein the front end of the dust collecting unit (1) is tapered (24) towards the inside.

9. Device as claimed in any of the claims 5 to 8 wherein the length and the diameter of the part of the suction device (1) limited by the sieve insert (26) and the mesh size of the sieve insert (26) are adjusted in such a way that only particles that are larger than the mesh size reach the sample-tube (3).

10. Device as claimed in any of the claims 5 to 9 wherein a motor-driven dust generating circular brush (28) is arranged in front of the dust collecting unit (1).

**Revendications**

1. Dispositif pour déterminer la teneur en magnétite et en phosphore dans des minérais à l'aide d'une bobine de mesure (4) engendrant un champ magnétique pour déterminer la teneur en magnétite et un réceptacle à échantillons (3) qui est placé dans le noyau de la bobine de mesure (4), caractérisé en ce que l'état de remplissage minimum du réceptacle à échantillons (3) est plus haut que la bobine de mesure (4), en ce que pour déterminer la quantité de l'échantillon, il est prévu une autre bobine de mesure (5) dont a longueur est supérieure à la hauteur de remplissage maximale du réceptacle à échantillons (3) et en ce que le réceptacle à échantillons (3) est relié à un vase de réaction (8) pour effectuer une analyse du phosphore, et qui est en communication avec les réservoirs à réactif (10, 11).

2. Dispositif selon la revendication 1, caractérisé en ce que le montage à bobines se compose des bobines de mesure (4, 5) montées selon le principe d'un transducteur avec en plus des deux bobines de mesure (4, 5) une bobine primaire, la longueur de la bobine primaire étant supérieure à la hauteur de remplissage maximale du réceptacle à échantillons (3).

3. Dispositif selon la revendication 1 ou 2, caractérisé en ce qu'entre les réservoirs à réactif (10, 11) et le vase de réaction (8), on a prévu des dispositifs de dosage qui se composent de chambres de dosage (12, 13) et de soupapes correspondantes (14–17 et 29–32), et une dérivation est réalisable dans les réservoirs (10, 11) par des vannes (29, 32).

4. Dispositif selon l'une des revendications 1 à 3, caractérisé en ce qu'il comporte un microcalculateur (22) pour exploiter les résultats des mesures, microcalculateur qui assure en même temps la commande de la mise en œuvre du dispositif.

5. Dispositif selon l'une des revendications 1 à 4, caractérisé en ce que le réceptacle à échantillons (3) est en communication avec une installation de collecte de poussière (1) de préférence par une conduite (2), verticale, coudée.

6. Dispositif selon la revendication 5, caractérisé en ce que la conduite coudée (2) et/ou le réceptacle à échantillons (3) comportent une installation de chauffage.

7. Dispositif selon la revendication 5 ou 6, caractérisé en ce que l'installation de collecte de poussière (1) est munie d'un ajutage d'aspiration (25), latéral et d'un jeu de tamis (26) intérieurs, montés coaxialement, en étant fermés à l'avant par un préfiltre (23).

8. Dispositif selon l'une des revendications 5 à 7, caractérisé en ce que l'installation de collecte de poussière (1) se rétrécit de façon conique (24) vers l'intérieur, vers l'avant.

9. Dispositif selon l'une des revendications 5 à 8, caractérisé en ce que la longueur et le diamètre de la partie de l'installation de collecte de poussière (1), délimités par le jeu de filtres (26) sont déterminés en fonction de la dimension des mailles du jeu de filtres (26) de façon que des particules plus grandes que les mailles arrivent dans le réceptacle à échantillons (3).

10. Dispositif selon l'une des revendications 5 à 9, caractérisé en ce que l'installation de collecte de poussière est munie à l'avant d'un balai annulaire, entraîné par un moteur et générant de la poussière.

Figur 1

0 043 826

Figur 2

Figur 3

28

Turbine

1

0 043 826

Figur 4

0 043 826

13